(19) Europäisches Patentamt
European Patent Office
Office européen des brevets

(11) Veröffentlichungsnummer: **0 362 729 A1**

(12) # EUROPÄISCHE PATENTANMELDUNG

(21) Anmeldenummer: **89118141.4**

(22) Anmeldetag: **30.09.89**

(51) Int. Cl.5: **C09B 29/00 , D06P 1/12 , D06P 3/68 , C07C 217/84 , C07C 213/00**

(30) Priorität: **01.10.88 DE 3833479**

(43) Veröffentlichungstag der Anmeldung: **11.04.90 Patentblatt 90/15**

(84) Benannte Vertragsstaaten: **CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Hertel, Hasso, Dr.**
**Brunnenweg 10**
**D6052 Mühlheim am Main(DE)**

(54) **Verfahren zur Herstellung wasserunlöslicher Azofarbstoffe auf der Faser.**

(57) Gemäß den Färbeweisen der Eisfarbentechnik werden Fasermaterialien, wie beispielsweise Cellulosefasermaterialien, gefärbt, indem man das mit einer Kupplungskomponente grundierte Fasermaterial in wäßrigem Medium mit der Diazoniumverbindung einer Anilinverbindung der nachstehend angegebenen und definierten allgemeinen Formel (1) kuppelt, wobei die Kupplungsreaktion und Farbstoffbildung auf der Faser bei einem pH-Wert zwischen 3 und 10 durchgeführt wird.

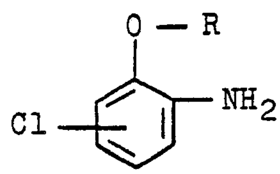
(1)

in welcher R eine geradkettige oder verzweigte Alkylgruppe von 3 bis 5 C-Atomen bedeutet und das Chlor in ortho- oder para-Stellung zur Alkoxygruppe gebunden ist.

EP 0 362 729 A1

## Verfahren zur Herstellung wasserunlöslicher Azofarbstoffe auf der Faser

Die vorliegende Erfindung liegt auf dem technischen Gebiet der Eisfarbenfärberei.

Die Herstellung von echten Färbungen nach den Methoden der Eisfarbentechnik durch Synthese von wasserunlöslichen Azofarbstoffen auf der Faser mittels der Reaktion einer Diazokomponente mit einer Kupplungskomponente ist in zahlreichen Veröffentlichungen seit langem beschrieben. Praktische Bedeutung hat vor allem die Bildung von Farbstoffen mit einer Kupplungskomponente aus der 2-Hydroxy-naphthalin-3-carbonsäurearylamid-Reihe erlangt. Unter den hierfür vorgeschlagenen Diazokomponenten ist auch 5-Chlor-2-methoxy-anilin (C.I. Azoic Diazo Component 10, C.I.-Nr. 37120). Diese Diazokomponente liefert mit 2-Hydroxy-naphthalin-3-carbonsäurearylamid-Verbindungen als Kupplungskomponenten auf der Faser rote Färbungen mit ausreichend guten Echtheiten. Jedoch war es wünschenswert, unter Verwendung von Diazokomponenten ähnlicher Konstitution verbesserte Färbungen zu erlangen.

Es wurde nun gefunden, daß man auf der Faser nach den Methoden der Eisfarbentechnik Färbungen mit verbesserten Eigenschaften, insbesondere besseren Naßechtheiten, herstellen kann, wenn man als Diazokomponente eine Anilinverbindung entsprechend der allgemeinen Formel (1)

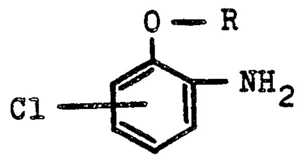

(1)

verwendet, in welcher R eine geradkettige oder verzweigte Alkylgruppe von 3 bis 5 C-Atomen bedeutet und das Chlor in ortho- oder para-Stellung zur Alkoxygruppe gebunden ist.

Die vorliegende Erfindung betrifft somit ein Verfahren zur Herstellung von wasserunlöslichen Azofarbstoffen auf der Faser, insbesondere auf der Cellulosefaser, nach den Methoden der Eisfarbentechnik, nach welcher man das mit einer Kupplungskomponente grundierte Fasermaterial in wäßrigem Medium mit der Diazoniumverbindung eines aromatischen Amins zusammenbringt und kuppelt, das dadurch gekennzeichnet ist, daß man als Diazokomponente eine Verbindung der obengenannten und definierten allgemeinen Formel (1) mit einer in der Eisfarbentechnik üblichen Kupplungskomponente, insbesondere einer aus der 2-Hydroxy-naphthalin-3-carbonsäurearylid-Reihe, kuppelt, wobei die Kupplungsreaktion und Farbstoffbildung auf der Faser bei einem pH-Wert zwischen 3 und 10, vorzugsweise zwischen 4 und 6, in wäßrigem Medium durchgeführt wird.

Als Kupplungskomponente dienen bevorzugt Verbindungen entsprechend der allgemeinen Formel (2)

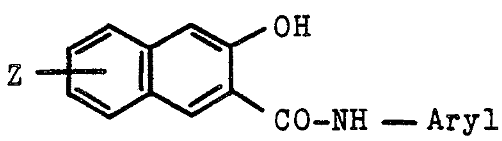

(2)

in welcher Z bevorzugt in 6- oder 7-Stellung gebunden ist und für ein Wasserstoffatom oder ein Halogenatom, wie ein Bromatom, oder eine Alkoxygruppe von 1 bis 4 C-Atomen, wie die Methoxy- oder Ethoxygruppe, steht und Aryl einen Phenylrest oder einen 1-Naphthylrest bedeutet, die beide durch 1, 2 oder 3 Substituenten aus der Gruppe Halogen, wie Brom und insbesondere Chlor, Nitro, Alkyl von 1 bis 4 C-Atomen, wie Ethyl und insbesondere Methyl, und Alkoxy von 1 bis 4 C-Atomen, wie Ethoxy und insbesondere Methoxy, substituiert sein können.

In den Verbindungen der allgemeinen Formel (1) ist R beispielsweise eine n-Propyl-, 1-Methyl-ethyl-, n-Butyl-, 1-Methyl-propyl-, 2-Methyl-propyl-, 1,1-Dimethyl-ethyl- oder n-Pentyl-Gruppe. Vorzugsweise ist R die n-Propyl-, n-Butyl-, n-Pentyl-, 1-Methyl-ethyl- oder 2-Methyl-propyl-Gruppe.

Verbindungen der allgemeinen Formel (1) sind beispielsweise 3-Chlor-2-(n-propoxy)-anilin, 3-Chlor-2-(n-butoxy)-anilin, 3-Chlor-2-(isopropoxy-anilin, 3-Chlor-2-(1-methyl-propoxy)-anilin, 3-Chlor-2-(2-methyl-propoxy)-anilin, 3-Chlor-2-(1,1-dimethyl-ethoxy)-anilin und 3-Chlor-2-(n-pentoxy)anilin sowie die entsprechenden 5-Chlor-Isomeren.

Kupplungskomponenten, wie beispielsweise solche der allgemeinen Formel (2), die in das erfindungsgemäße Verfahren eingesetzt werden könnnen, sind beispielsweise: das Phenylamid, das 2-Methylphenylamid, das 2-Ethylphenylamid, das 2-Methoxyphenylamid, das 4-Methoxyphenylamid, das 2-Ethoxyphenylamid, das 4-Chlorphenylamid, das 4-Chlor-2-methyl-phenylamid, das 3-Nitrophenylamid, das 5-Chlor-2-methoxy-phenylamid, das 5-Chlor-2-methyl-phenylamid, das 4-Methoxy-2-methyl-phenylamid, das 2,5-Dimethoxyphenylamid, das 5-Chlor-2,4-dimethoxy-phenylamid, das 4-Chlor-2,5-dimethoxy-phenylamid, das 4-Chlor-2-methoxy-5-methyl-phenylamid, das 5-Brom-2-methoxy-phenylamid und das Naphth-1-yl-amid der 2-Hydroxy-naphthalin-3-carbonsäure, das 6-Brom-2-hydroxy-naphthalin-3-carbonsäure-(2-methoxyphenyl)-amid, 6-Methoxy-2-hydroxy-naphthalin-3-carbonsäure-phenylamid und 6-Methoxy-2-hydroxy-naphthalin-3-carbonsäure-(4-chlor-2,5-dimethoxy-phenyl)-amid.

In den in dem erfindungsgemäßen Verfahren verwendbaren Kupplungskomponenten der allgemeinen Formel (2) bedeutet Aryl bevorzugt den Phenyl-, 2-Methyl-phenyl-, 2-Methoxy-phenyl-, 4-Methoxy-phenyl-, 2-Ethoxy-phenyl-, 4-Ethoxy-phenyl-, 4-Methoxy-2-methyl-phenyl-, 2,5-Dimethoxyphenyl-, 4-Chlor-phenyl-, 4-Chlor-2-methyl-phenyl-, 5-Chlor-2-methyl-phenyl-, 5-Chlor-2-methoxy-phenyl-, 4-Chlor-2-methoxy-5-methyl-phenyl-, 4-Chlor-2,5-dimethoxy-phenyl-, 5-Chlor-2,4-dimethoxy-phenyl und den 1-Naphthyl-Rest.

Die Bildung der Azofarbstoffe unter der erfindungsgemäßen Verwendung der Diazokomponente entsprechend den allgemeinen Formeln (1) erfolgt gemäß den Verfahrensweisen der Eisfarbentechnik, die in der Literatur vielfach beschrieben sind (s. bspw. H. Rath, Lehrbuch der Textilchemie, 3. Aufl., Springer Verlag (1972), Seiten 548 ff., und K. Venkataraman, The Chemistry of Synthetic Dyes, Vol. I, Academic Press, New York (1952), Seiten 650-704), so beispielsweise analog bekannten Verfahrensweisen der Stückfärbung in Kontinueverfahren, der Kreuzspulfärbung nach dem Ausziehverfahren und des Textildrucks.

Beispielsweise kann man so vorgehen, daß man ein Fasermaterial, insbesondere ein Cellulosefasermaterial, das zuvor mit der Kupplungskomponente alkalisch vorgrundiert wurde, mit der Diazoniumverbindung des Anilins entsprechend der allgemeinen Formel (1) in wäßrigem Medium zusammenbringt. Dies kann allgemein so geschehen, daß man analog üblicher Methoden der Eisfarbentechnik das mit einer alkalischen wäßrigen Lösung der Kupplungskomponente grundierte Material mit einer getrennt hergestellten wäßrigen Lösung des Diazoniumsalzes, die so viel sauer-wirkende Mittel und/oder Puffersubstanzen enthält, daß sich ein pH-Bereich zwischen 3 und 10, vorzugsweise zwischen 4 und 6, einstellt und die Kupplungsreaktion sodann innerhalb dieses pH-Bereiches abläuft, zusammenbringt. Man kann aber auch die Chlor-alkoxy-anilin-Verbindung in wäßrigem Medium in Gegenwart des grundierten Materials diazotieren, wobei gleichzeitig oder anschließend die Kupplungsreaktion durchgeführt wird; für diese Variante wird entweder die Diazotierung im schwach sauren Medium durchgeführt oder anschließend an die Diazotierung der pH auf einen für die Kupplungsreaktion günstigen Wert zwischen 3 und 10 gebracht.

Für alle diese Durchführungsformen wird zweckmäßig die Menge der bei der Diazotierung verwendeten Säure und/oder die Menge des Alkalis im grundierten Material bzw. des wäßrigen Mediums, in welchem das zu färbende, grundierte Material vorliegt, bereits zuvor so bemessen, daß, erforderlichenfalls durch Zusatz von Puffersubstanzen oder Puffergemischen, der erwünschte pH-Wert für die durchzuführende Kupplung sich gleich einzustellen vermag.

Die Diazotierung der Verbindungen (1) erfolgt bei den in der Eisfarbentechnik üblichen Temperaturen, beispielsweise bei einer Temperatur zwischen -5 °C und +35 °C, bevorzugt 0 bis 10 °C.

Im einzelnen kann man beispielsweise wie folgt vorgehen: Die Chlor-alkoxy-anilin-Verbindung der Formel (1) wird, vorzugsweise in verdünnter wäßriger Salzsäure, mittels einer wäßrigen Natriumnitritlösung diazotiert; danach wird überschüssige Mineralsäure mit einem säurebindenden Mittel, wie beispielsweise Natriumacetat, Natriumbicarbonat oder Natriumphosphat, gebunden und die wäßrige Diazoniumsalz-Lösung anschließend, gegebenenfalls nach Zugabe eines alkalibindenden Mittels, wie Essigsäure, Natriumdihydrogenphosphat, Borsäure oder Chromacetat, als Färbeflotte oder Färbebad verwendet. Dieses kann mittels eines Foulards auf das zu färbende Material, hier insbesondere ein Gewebe, das zuvor mit dem Alkalisalz der Kupplungskomponente imprägniert und getrocknet worden war, aufgebracht werden. Man kann diese Flotte auch in der Weise verwenden, daß man das imprägnierte, zu färbende Material, wie in Form eines Gewebes oder in Form von Fäden oder Garnen, in diese wäßrige Diazoniumsalz-Lösung als Färbebad einbringt und es darin bewegt, sei es per Hand oder sei es durch oder in entsprechende(n) in der Technik übliche(n) Apparate(n), wie Haspelkufe, Jigger oder eine(r) Jet-Apparatur. Hierbei wird der erforderliche Kupplungsbereich zwischen 3 und 10 durch entsprechende säurebindende oder alkalibindende Mittel oder Puffersubstanzen eingestellt und gehalten.

Bei der Diazotierung kann anstelle von verdünnter wäßriger Salzsäure auch verdünnte wäßrige Schwefelsäure verwendet werden, ebenfalls eine verdünnte wäßrige mittelstarke Säure, wie Phosphorsäure, Chloressigsäure, Milchsäure, Glykolsäure oder Ameisensäure. Die Diazotierung kann auch in einer wäßrigen Phase vorgenommen werden, bei der die Viskosität mittels einer üblichen Druckverdickung erhöht wurde.

Eine andere Variante der Farbstoffbildung kann nach einem Druckverfahren erfolgen, indem man die

wäßrige Lösung des Diazoniumsalzes mit einem geeigneten und in der Technik üblichen Mittel, wie Stärke, Traganth, einem Celluloseether oder Celluloseester, verdickt und ein mit dem Alkalisalz der Kupplungskomponente imprägniertes Gewebe mit dieser verdickten Lösung bedruckt, wobei man auch hier einen entsprechenden Kupplungs-pH-Wert zwischen 3 und 10 durch entsprechende Wahl der säurebindenden oder alkalibindenden Mittel oder Puffersubstanzen und mit den hierfür benötigten Mengen einstellt und aufrechterhält.

Eine andere Variante des erfindungsgemäßen Verfahrens zur Herstellung von Azofarbstoffen mit einer Diazokomponente der Formel (1) und einer entsprechenden Kupplungskomponente auf der Faser besteht darin, daß man eine verdickte, zusätzlich eine schwache oder mittelstarke Säure enthaltende Emulsion oder Dispersion der Anilinverbindung der Formel (1) auf das zu färbende Gewebe, das zuvor mit dem Alkalisalz der Kupplungskomponente und Natriumnitrit imprägniert worden war, aufdruckt.

Das gefärbte Material wird anschließend, wie in der Eisfarbentechnik üblich, durch gründliches Spülen mit Wasser, alkalisches Seifen, nochmaliges Spülen mit Wasser und Trocknen fertiggestellt. Die erfindungsgemäß erhältlichen Färbungen zeigen gute Echtheitseigenschaften, insbesondere sehr gute Naßechtheiten, von denen insbesondere die Waschechtheiten bei verschiedenen Temperaturen, die Peroxidwaschechtheit, die Wasserstoffperoxidechtheit, die Sodakochechtheit, die Mercerisierechtheit, die Bleich-, Trockenreinigungs- und Reibechtheit hervorgehoben werden können.

Die Verbindungen der allgemeinen Formel (1) sind teilweise in der Literatur beschrieben. Die noch nicht beschriebenen, neuen Verbindungen der Formel (1) lassen sich analog den bekannten Verfahrensweisen, nach welchen die bekannten Verbindungen synthetisiert werden können, herstellen, so beispielsweise durch Umsetzung von 2,3-Dichlor- oder 2,5-Dichlor-nitrobenzol mit dem entsprechenden $C_3$-$C_5$-Alkanol in Gegenwart von einem Alkalihydroxid, wie Natriumhydroxid, und anschließende Reduktion der Nitrogruppe. Beispielsweise verfährt man in der Weise, daß man das Dichlor-nitrobenzol in etwa 25 bis 60 gew.-%iger wäßriger Natronlauge bei erhöhter Temperatur, wie etwa bei einer Temperatur zwischen 50 und 90 °C, mit dem Alkanol, zweckmäßig in Gegenwart eines Phasentransfer-Katalysators, wie beispielsweise einem Tetraalkylammoniumsalz, umsetzt, beispielsweise indem man zu dem Ansatz aus dem Dichlor-nitrobenzol in der wäßrigen Natronlauge das Alkanol langsam zugibt. Man rührt noch mehrere Stunden nach und trennt sodann die organische Phase ab. Das darin enthaltene überschüssige Alkanol wird abdestilliert und das erhaltene rohe Chlor-nitro-alkoxybenzol in üblicher Weise reduziert, beispielsweise durch katalytische Hydrierung (wie beispielsweise mit einem üblichen Nickel-oder Palladium-Katalysator) bei einem Wasserstoffdruck von 1 bis 100 bar, vorzugsweise 40 bis 80 bar, und einer Hydriertemperatur von 20 bis 120 °C, vorzugsweise 40 bis 70 °C, wobei ein niederes Alkanol, vorzugsweise Methanol, als Löse- oder Verdünnungsmittel zugegen sein kann. Die Reduktion kann auch nach Béchamp in wäßrigem Medium mittels Eisenpulver und eine Säure in katalytischer Menge, wie beispielsweise Salzsäure, Ameisensäure oder Essigsäure, wobei auch ein aromatischer Kohlenwasserstoff als Lösemittel oder Verdünnungsmittel, wie beispielsweise Xylol, zugegen sein kann. Das nach Reduktion isolierte Chlor-alkoxy-anilin kann sodann durch fraktionierte Destillation unter vermindertem Druck in reiner Form isoliert werden.

Das Zwischenprodukt Chlor-nitro-alkoxy-benzol kann auch erhalten werden, indem man 4-Chlor- oder 6-Chlor-2-nitrophenol in alkalischer, wäßriger oder alkanolischer Lösung mit einem dem Rest R entsprechenden Halogenalkan, vorzugsweise einem Bromalkan, bei einer Temperatur zwischen 50 und 150 °C umsetzt. Es wird anschließend nach Zugabe von Wasser durch Abtrennung der organischen Phase und Abdestillieren der leichtflüchtigen Bestandteile isoliert.

Die vorliegende Erfindung betrifft deshalb auch neue Verbindungen entsprechend der allgemeinen Formel (1A) und (1B)

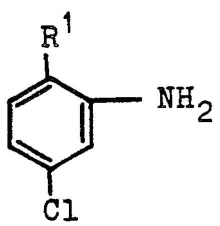
(1A)

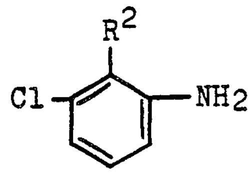
(1B)

in welchen R1 für die n-Propoxy-, die 1-Methyl-ethoxy-, die 2-Methyl-butoxy- oder die Pentoxy-Gruppe steht und $R^2$ die n-Propoxy-, die 1-Methyl-ethoxy, die n-Butoxy-, die 2-Methyl-propoxy-, die 2-Methylbutoxy-, die 3-Methylbutoxy- und die n-Pentoxy-Gruppe bedeutet, sowie ein Verfahren zur Herstellung dieser Verbindungen durch Umsetzung von 2,5-Dichlor-nitrobenzol mit n-Propanol, Isopropanol, 2-Methyl-butanol oder n-Pentanol bzw. von 2,3-Dichlor-nitrobenzol mit n-Propanol, Isopropanol, n-Butanol, 2-Methyl-propanol, 2-Methyl-butanol, 3-Methylbutanol oder n-Pentanol oder durch Umsetzung von 4-Chlor- oder 6-Chlor-2-nitrophenol mit dem entsprechenden Halogenalkan der Formel Hal-$R^1$ bzw. Hal-$R^2$ in Gegenwart eines Alkali und anschließende Reduktion der Nitrogruppe der erhaltenen 5-Chlor- oder 3-Chlor-2-alkoxy-nitrobenzol-Verbindung, beispielsweise analog den obigen Verfahrensangaben. Desweiteren betrifft die vorliegende Erfindung die Verwendung dieser neuen Verbindungen zur Herstellung von wasserunlöslichen Azofarbstoffen, insbesondere auf der Faser, vorzugsweise Cellulosefaser, nach den Methoden der Eisfarbentechnik.

Die erfindungsgemäß eingesetzten Anilinverbindungen zeichnen sich durch ein niedriges mutagenes Potential aus. So verhält sich 3-Chlor-2-(n-butoxy)-anilin im sogenannten Ames-Test (s. B.N. Ames et al., Proc. Nat. Acad. Sci. USA 70, 2201-2205 (1973); B.N. Ames et al., Mutation Res. 31, 347-364 (1975)) negativ.

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

**Färbebeispiel 1**

Zur Herstellung einer Färbung eines Baumwollgewebes imprägniert man das Gewebe zunächst mit einer wäßrigen Grundierflotte, bestehend aus einer Lösung von 20 Teilen 2-Hydroxy-naphthalin-3-carbonsäure(2′-methyl-phenyl)amid, 8,3 Teilen Natriumhydroxid und 5 Teilen eines handelsüblichen Schutzkolloides in 1000 Teilen Wasser. Die Flottenaufnahme soll 70 % des Warengewichtes betragen. Das imprägnierte Gewebe wird bei etwa 130 °C getrocknet und anschließend mit einer das Diazoniumsalz des 3-Chlor-2-(n-butoxy)-anilins enthaltenden wäßrigen Entwicklungsflotte bei einer Flottenaufnahme von 70 %, bezogen auf das Warengewicht überklotzt. Die Entwicklungsflotte wird durch Diazotieren von 15,8 Teilen 3-Chlor-2-(n-butoxy)-anilin in 215 Teilen einer 4%igen wäßrigen Salzsäure mittels 15 Teilen einer 40 %igen wäßrigen Natriumnitritlösung und nachfolgende Zugabe von 13 Teilen Natriumacetat-trihydrat und 10 Teilen Essigsäure und Auffüllen der erhaltenen Diazoniumsalzlösung mit Wasser auf 1000 Teile hergestellt.

Nach dem Überklotzen mit der Entwicklungsflotte erfolgt ein Luftgang des überklotzten Gewebes von etwa 1 Minute und anschließend eine Heißwasserpassage. Die Nachbehandlung der erhaltenen Färbung erfolgt in üblicher Weise, beispielsweise durch Spülen des gefärbten Gewebes in kaltem und heißem Wasser, durch kochende Behandlung während etwa 15 Minuten in einem ein nichtionogenes Waschmittel enthaltenden Bad, durch erneutes Spülen in heißem und kaltem Wasser und Trocknen.

Man erhält eine Orangefärbung von sehr guten Echtheitseigenschaften, von denen insbesondere die guten Naßechtheitseigenschaften hervorgehoben werden können.

**Färbebeispiel 2**

Zur Herstellung einer Färbung verfährt man in der im Färbebeispiel 1 angegebenen Weise, setzt jedoch anstelle von 3-Chlor-2-(n-butoxy)-anilin als Diazokomponente in der Entwicklungsflotte das isomere 5-Chlor-2-(n-butoxy)-anilin ein. Man erhält eine Rotfärbung von sehr guten Echtheitseigenschaften, von denen ebenfalls insbesondere die guten Naßechtheitseigenschaften hervorgehoben werden können.

**Färbebeispiel 3**

a) Es wird zunächst die folgende Grundierungsflotte hergestellt: 15,3 Teile 2-Hydroxy-naphthalin-3-carbonsäure(5′-chlor-2′-methoxy-phenyl)-amid werden mit 36 Teilen Ethanol angeteigt und durch Zugabe von 10,6 Teilen einer 32%igen wäßrigen Natronlauge sowie 23 Teilen Wasser von 40 °C gelöst. 7,6 Teile einer 33%igen wäßrigen Formaldehydlösung werden zugegeben, und die so bereitete Stammlösung wird nach 10 Minuten in eine 35 °C warme Lösung von 58 Teilen einer 32%igen wäßrigen Natronlauge, 120 Teilen Natriumchlorid und 18 Teilen eines handelsüblichen Fettsäure-Eiweißabbauprodukt-Kondensationsproduktes in 6000 Teilen weichem Wasser eingerührt. Mit dieser Grundierungsflotte wird in einem Kreuzspulapparat eine Kreuzspule mit 600 Teilen Baumwollgarn, das zur Entfernung von störenden Nichtcellulose-Substanzen alkalisch mit einem Tensid und mit Sequestriermittel abgekocht worden war, 30 Minuten lang durchflossen. Anschließend wird die Grundierungsflotte abgelassen und die grundierte Spule 10 Minuten

lang mit einer Lösung von 240 Teilen Natriumchlorid und 4 Teilen einer 32%igen wäßrigen Natronlauge in 6000 Teilen Wasser gespült. Das Spülbad wird anschließend abgelassen.

b) Sodann wird dem Kreuzspulapparat eine Entwicklungsflotte zugeführt, die wie folgt bereitet wird: 17,2 Teile 5-Chlor-2-(n-propoxy-)anilin werden unter gutem Rühren in eine Mischung aus 100 Teilen Wasser und 29 Teilen einer 32%igen Salzsäure eingerührt. Etwa 30 Teile Eis werden zugesetzt; sodann wird das Amin unter gutem Rühren durch allmähliche Zugabe von 6,9 Teilen Natriumnitrit in konzentriert-wäßriger Lösung diazotiert. Man rührt 30 Minuten nach und gießt diese Stammlösung sodann in eine Lösung aus 4 Teilen Essigsäure, 70 Teilen Natriumacetat-trihydrat und 12 Teilen eines Octadecylalkohol-polyglykolethers in 5800 Teilen Wasser ein. Diese Entwicklungsflotte wird 30 Minuten lang bei 20° C durch die grundierte Kreuzspule gepumpt und anschließend aus dem Apparat abgelassen. Die Kreuzspule wird sodann in üblicher Weise mit einer essigsauren wäßrigen Lösung gespült, sodann mit kaltem Wasser klargespült und zunächst bei 60° C, dann bei 100° C geseift, anschließend mit warmem und kaltem Wasser gespült und schließlich getrocknet.

Es wird eine rote Baumwollfärbung in guter Farbausbeute und mit guten Echtheiten, wie insbesondere Waschechtheiten, erhalten.

**Färbebeispiel 4**

a) 3,5 Teile 2-Hydroxy-naphthalin-3-carbonsäure-(naphth-1′-yl)-amid werden mit einer etwa 35° C warmen Mischung aus 4,5 Teilen Ethanol, 5,5 Teilen Wasser, 2,4 Teilen einer 32 %igen wäßrigen Natronlauge und 2 Teilen einer 33%igen wäßrigen Formaldehydlösung übergossen und durch Rühren gelöst. Nach etwa 10 Minuten wird diese Lösung in 1000 Teile Wasser, die 8,5 Teile einer 32,5%igen wäßrigen Natronlauge und 2 Teile eines Fettsäure-Eiweißabbauprodukt-Kondensationsproduktes gelöst enthalten, gegeben. In das so hergestellte Bad trägt man 50 Teile eines gebleichten, abgekochten und vorgenetzten Baumwollgarnes ein und bewegt es darin 30 Minuten. Es wird sodann herausgenommen und in einer Lösung aus 30 Teilen Natriumchlorid und 1,3 Teilen einer 32%igen wäßrigen Natronlauge in 1000 Teilen Wasser gespült.

b) Getrennt hiervon wird das Entwicklungsbad wie folgt hergestellt: 2,1 Teile 5-Chlor-2-(2′-methyl-propoxy)anilin werden in ein Gemisch aus 20 Teilen Wasser und 3,2 Teilen einer wäßrigen 32%igen Salzsäure eingerührt. Nach einigem Nachrühren gibt man 5 Teile Eis hinzu und diazotiert mit 0,72 Teilen Natriumnitrit in konzentriert-wäßriger Lösung. Nach Beendigung der Diazotierung wird die Lösung mit Wasser auf 1000 Teile verdünnt, und es werden noch 10 Teile Natriumacetattrihydrat, 0,2 Teile Essigsäure und 1 Teil eines Alkylpolyglykolethers darin gelöst.

c) Zur Herstellung der Färbung auf dem Textilmaterial verfährt man beispielsweise wie folgt: Das gemäß a) grundierte, gespülte und von der überschüssigen Flotte durch Abquetschen befreite Garn wird in das unter b) beschriebene Entwicklungsbad eingebracht und darin 30 Minuten bei etwa 20° C behandelt. Anschließend wird das Material herausgenommen, wie üblich gespült, geseift, nochmals gespült und getrocknet.

Es wird eine rote Färbung in guter Farbausbeute mit guten Echtheiten, insbesondere sehr guten Waschechtheiten, erhalten.

**Färbebeispiel 5**

Zur Herstellung einer Färbung verfährt man gemäß der Verfahrensweise des Färbebeispieles 4, setzt jedoch als Diazokomponente in dem Entwicklungsbad anstelle des 5-Chlor-2-(2′-methyl-propoxy)-anilin das 5-Chlor-2-(n-pentoxy)anilin in äquivalenter Menge ein. Man erhält ebenfalls in guter Farbausbeute eine rote Färbung mit guten Echtheitseigenschaften, von denen insbesondere die sehr guten Waschechtheiten hervorgehoben werden können.

**Färbebeispiel 6**

Zur Herstellung eines roten Druckmusters verfährt man beispielsweise wie folgt: Man grundiert ein Baumwollgewebe mit 2-Hydroxy-naphthalin-3-carbonsäurephenylamid gemäß den Angaben des Färbebeispieles 1, trocknet es und bedruckt es auf einer Film- oder Rouleaux-Druckmaschine mit einer Druckpaste, die wie folgt zubereitet wird:

In 600 Teile einer üblichen wäßrigen Druckverdickung werden 5,4 Teile Natriumnitrit und anschließend 17,5 Teile einer Lösung, bestehend zu 50 % aus 3-Chlor-2-(n-propoxy)-anilin, zu 25 %aus Ethylendiglykol und zu 25 % aus einem Umsetzungsprodukt von Ricinusöl mit 36 Mol Ethylenoxid, eingerührt. Gesondert hiervon werden in 300 Teile einer üblichen wäßrigen Druckverdickung 18 Teile einer 85%igen wäßrigen Phosphorsäure eingerührt. Die beiden Verdickungen werden sodann gut vermischt und mit Wasser oder einer Druckverdickung auf 1000 Teile aufgefüllt.

Das mit dieser Druckpaste bedruckte Gewebe wird in üblicher Weise, wie beispielsweise in den vorherigen Färbebeispielen erwähnt, nachbehandelt und fertiggestellt. Es wird ein kräftiges orangerotes Druckmuster mit guten Echtheitseigenschaften erhalten.

Alternativ kann man die Druckpaste auch wie folgt bereiten: 8,5 Teile 3-Chlor-2-(n-butoxy)-anilin werden in ein Gemisch aus 100 Teilen Wasser und 21 Teilen einer 32%igen wäßrigen Salzsäure eingerührt und bei einer Temperatur von etwa 5°C mit 9 Teilen einer 40%igen wäßrigen Natriumnitritlösung diazotiert. 6 Teile Essigsäure und 9 Teile Natriumacetattrihydrat werden noch zugegeben, und das Ganze wird mit Wasser und Verdickung auf 1000 Teile aufgefüllt.

**Färbebeispiel 7**

Man verfährt gemäß der Verfahrensweise des Färbebeispieles 1, ersetzt jedoch das 2-Hydroxy-naphthalin-3-carbonsäure- (2′-methyl-phenyl)-amid durch die äquivalente Menge an 2-Hydroxy-naphthalin-3-carbonsäure-phenylamid, 2-Hydroxy-naphthalin-3-carbonsäure-(4′-methoxy-phenyl)-amid, 2-Hydroxy-naphthalin-3-carbonsäure-(4′-methoxy-2′-methylphenyl)-amid, 2-Hydroxy-naphthalin-3-carbonsäure-(2′-methoxy-phenyl)-amid, 2-Hydroxy-naphthalin-3-carbonsäure-(2′,5′-dimethoxy-phenyl)-amid, 2-Hydroxy-naphthalin-3-carbonsäure-(3′-nitro-phenyl)-amid, 2-Hydroxy-naphthalin-3-carbonsäure-(5′-chlor-2′-methyl-phenyl)-amid oder 2-Hydroxy-naphthalin-3-carbonsäure-(2′-ethoxy-phenyl)-amid. Man erhält orangerote Färbungen mit guten Echtheitseigenschaften.

**Färbebeispiel 8**

Man verfährt gemäß der Verfahrensweise des Färbebeispieles 1, setzt jedoch anstelle von 3-Chlor-2-(n-butoxy)-anilin die gleiche Menge an 5-Chlor-2-(1′-methyl-propoxy)-anilin oder die äquivalente Menge an 5-Chlor-2-(n-propoxy)-anilin ein. Man erhält rote Cellulosefaserfärbungen mit guten Echtheitseigenschaften.

**Färbebeispiel 9**

Man verfährt gemäß der Verfahrensweise des Färbebeispieles 4, setzt jedoch anstelle von 3,5 Teilen 2-Hydroxy-naphthalin-3-carbonsäure-(naphth-1′-yl)-amid 3,0 Teile 2-Hydroxy-naphthalin-3-carbonsäure-(5′-chlor-2′,4′-dimethoxy-phenyl)-amid oder 1,8 Teile 2-Hydroxy-naphthalin-3-carbonsäure-(4′-chlor-2′-methoxy-5′-methyl-phenyl)-amid oder 1,6-Teile 2-Hydroxy-naphthalin-3-carbonsäure-(4′-chlor-2′,5′-dimethoxy-phenyl)-amid ein. Man erhält rote Färbungen des Cellulosefasermaterials mit guten Echtheitseigenschaften.

**Färbebeispiel 10**

Man verfährt gemäß der Verfahrensweise des Färbebeispieles 4 oder 9, setzt jedoch anstelle von 5-Chlor-2-(2′-methyl-propoxy)-anilin die gleiche Menge an 3-Chlor-2-(2′-methyl-propoxy)-anilin ein. Man erhält orangerote Färbungen des Cellulosefasermaterials mit guten Echtheitseigenschaften.

**Färbebeispiel 11**

Man verfährt gemäß der Verfahrensweise des Färbebeispieles 4 oder 9, setzt jedoch anstelle von 5-Chlor-2-(2′-methyl-propoxy)-anilin die äquivalente Menge an 5-Chlor-2-(n-propoxy)-anilin ein. Man erhält rote Färbungen des Cellulosefasermaterials mit guten Echtheitseigenschaften.

**Beispiel A**

192 Teile 2,5-Dichlor-1-nitro-benzol, 60 Teile Wasser und 95 Teile Natriumhydroxyd werden auf 70 °C erwärmt. Innerhalb von 4 Stunden gibt man eine Lösung von 26 Teilen Tetrabutylammonium-bromid in 93 Teilen n-Propanol hinzu. Man rührt bei 70 °C noch drei Stunden nach und versetzt den Ansatz nach Abkühlen auf 25 °C mit 400 Teilen Wasser. Die organische Phase wird abgetrennt und zweimal mit jeweils 400 Teilen Wasser und anschließend mit 400 Teilen einer wäßrigen 1%igen Salzsäure ausgerührt.

Das erhaltene rohe 4-Chlor-2-nitro-1-propoxy-benzol wird innerhalb einer Stunde in ein siedendes Gemisch aus 100 Teilen Wasser, 200 Teilen Xylol und 5 Teilen Ameisensäure sowie 300 Teilen Eisenpulver eingetragen. Man hält den Ansatz noch eine Stunde bei Siedetemperatur, kühlt ihn sodann auf etwa 50 °C ab, setzt bis zur schwach alkalischen Reaktion Natriumcarbonat hinzu, filtriert von dem Eisenschlamm ab und entfernt zunächst das Xylol durch Destillation bei etwa 50 mbar.

Bei der anschließenden Destillation unter stark reduziertem Druck erhält man das 5-Chlor-2-propoxy-anilin in guter Reinheit:
Sdp.: 81 °C/0,1 mbar; Smp.: -1 °C.

**Beispiel B**

Man verfährt gemäß der Verfahrensweise von Beispiel A, verwendet jedoch anstelle von n-Propanol die gleiche Menge an iso-Propanol. Man erhält das 5-Chlor-2-(1-methyl-äthyl)-anilin mit einem Siedepunkt von 77 °C bei 0,1 mbar.

**Beispiel C**

Man verfährt gemäß der Verfahrensweise des Beispieles A, verwendet jedoch anstelle von n-Propanol die äquivalente Menge an n-Pentanol. Man erhält das 5-Chlor-2-pentoxy-anilin mit einem Siedepunkt von 101 °C bei 0,1 mbar und einem Schmelzpunkt von -4 °C.

**Beispiel D**

Man verfährt gemäß der Verfahrensweise des Beispieles A, verwendet jedoch anstelle von n-Propanol die äquivalente Menge an 2-Methyl-butanol. Man erhält das 5-Chlor-2-(2-methyl-butoxy)-anilin mit einem Siedepunkt von 93 °C bei 0,1 mbar.

**Beispiel E**

Man verfährt gemäß der Verfahrensweise des Beispieles A, verwendet jedoch anstelle von 2,5-Dichlor-nitro-benzol das 2,3-Dichlor-nitro-benzol in gleicher Menge. Man erhält das 3-Chlor-2-propoxy-anilin mit einem Siedepunkt 84 °C bei 0,3 mbar

**Beispiel F**

Man verfährt gemäß der Verfahrensweise von Beispiel E, verwendet jedoch anstelle von n-Propanol die äquivalente Menge an n-Butanol. Man erhält das 3-Chlor-2-butoxy-anilin bei einem Siedepunkt von 82 °C/0,05 mbar.

**Beispiel G**

Man verfährt gemäß der Verfahrensweise von Beispiel E, verwendet jedoch anstelle von n-Propanol die äquivalente Menge an n-Pentanol. Man erhält das 3-Chlor-2-pentoxy-anilin bei einem Siedepunkt von 98 °C/0,1mbar

**Beispiel H**

Man verfährt gemäß der Verfahrensweise von Beispiel E, verwendet jedoch anstelle von n-Propanol die äquivalente Menge an 1-Methyl-äthanol. Man erhält das 3-Chlor-2-(1-methyl-äthoxy)-anilin bei einem Siedepunkt von 75° C/0,3 mbar.

**Beispiel J**

Man verfährt gemäß der Verfahrensweise von Beispiel E, verwendet jedoch anstelle von n-Propanol die äquivalente Menge an 2-Methyl-propanol. Man erhält das 3-Chlor-2-methyl-propoxy-anilin bei einem Siedepunkt von 88° C/0,1 mbar.

## Ansprüche

1. Verfahren zur Herstellung von wasserunlöslichen Azofarbstoffen auf der Faser, insbesondere auf der Cellulosefaser, nach den Methoden der Eisfarbentechnik, nach welcher man das mit einer Kupplungskomponente grundierte Fasermaterial in wäßrigem Medium mit der Diazoniumverbindung eines aromatischen Amins zusammenbringt und kuppelt, dadurch gekennzeichnet, daß man als diazotierbare Diazokomponente eine Anilinverbindung der allgemeinen Formel (1)

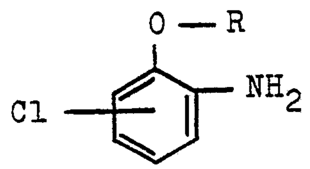

(1)

einsetzt, in welcher H eine geradkettige oder verzweigte Alkylgruppe von 3 bis 5 C-Atomen bedeutet und das Chlor in ortho- oder para-Stellung zur Alkoxygruppe gebunden ist, und mit einer in der Eisfarbentechnik üblichen Kupplungskomponente kuppelt und hierbei die Kupplungsreaktion und Farbstoffbildung auf der Faser bei einem pH-Wert zwischen 3 und 10 durchführt.

2. Verfahren nach Anspruch 1, dadurch gekennzeichnet, daß die Kupplungskomponente eine Verbindung entsprechend der allgemeinen Formel (2)

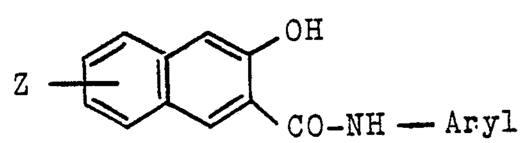

(2)

ist, in welcher Z für ein Wasserstoffatom oder ein Halogenatom oder eine Alkoxygruppe von 1 bis 4 C-Atomen steht und Aryl einen Phenylrest oder einen 1-Naphthylrest bedeutet, die beide durch 1, 2 oder 3 Substituenten aus der Gruppe Halogen, Nitro, Alkyl von 1 bis 4 C-Atomen, und Alkoxy von 1 bis 4 C-Atomen, substituiert sein können.

3. Verfahren nach Anspruch 1 oder 2, dadurch gekennzeichnet, daß man ein aromatisches Amin der allgemeinen Formel (1) einsetzt, in welcher R die n-Propyl-, n-Butyl-, n-Pentyl-, 1-Methyl-ethyl- oder 2-Methyl-propyl-Gruppe ist.

4. Verfahren nach mindestens einem der Ansprüche 1 bis 3, dadurch gekennzeichnet, daß in der verwendeten Kupplungskomponente der Formel (2) der Rest Aryl der Phenyl-, 2-Methyl-phenyl, 2-Methoxy-phenyl-, 4-Methoxy-phenyl-, 2-Ethoxy-phenyl-, 4-Ethoxy-phenyl-, 4-Methoxy-2-methyl-phenyl-, 2,5-Dimethoxy-phenyl-, 4-Chlor-phenyl-, 4-Chlor-2-methyl-phenyl-, 5-Chlor-2-methyl-phenyl, 5-Chlor-2-methoxy-phenyl, 4-Chlor-2-methoxy-5-methyl-phenyl, 4-Chlor-2,5-dimethoxy-phenyl-, 5-Chlor-2,4-dimethoxy-phenyl- oder der 1-Naphthyl-Rest ist.

5. Verbindung entsprechend der allgemeinen Formel (1A) oder (1B)

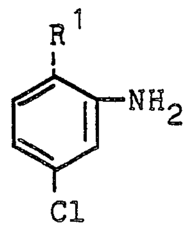

(1A)

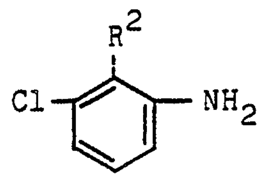

(1B)

in welchen $R^1$ für die n-Propoxy-, 1-Methyl-ethoxy-, 2-Methyl-butoxy oder n-Pentoxy-Gruppe steht und $R^2$ die n-Propoxy, die 1-Methyl-ethoxy, die n-Butoxy-, die 2-Methyl-Propoxy-, die 2-Methyl-butoxy-, die 3-Methyl-butoxy- und die n-Pentoxy-Gruppe bedeutet.

6. Verfahren zur Herstellung einer in Anspruch 5 genannten und definierten Verbindung der allgemeinen Formel (1A) oder (1B), dadurch gekennzeichnet, daß man 2,5-Dichlor-nitrobenzol mit n-Propanol, Isopropanol, 2-Methyl-butanol- oder n-Pentanol bzw. 2,3-Dichlor-nitrobenzol mit n-Propanol, Isopropanol, n-Butanol, 2-Methyl-propanol, 2-Methyl-butanol, 3-Methyl-butanol oder n-Pentanol in Gegenwart eines Alkalihydroxids umsetzt oder 4-Chlor- oder 6-Chlor-2-nitro-phenol mit dem entsprechenden Halogenalkan der Formel Hal-$R^1$ bzw. Hal-$R^2$ in Gegenwart eines Alkali umsetzt und daß man in der erhaltenen 5-Chlor- bzw. 3-Chlor-2-alkoxy-anilin-Verbindung die Nitrogruppe zur Aminogruppe reduziert.

7. Verbindung nach Anspruch 5 der allgemeinen Formel (1A), dadurch gekennzeichnet, daß $R^1$ die n-Propoxy-Gruppe bedeutet.

8. Verbindung nach Anspruch 5 der allgemeinen Formel (1B) dadurch gekennzeichnet, daß $R^2$ die n-Propoxy- oder die n-Butoxy-, oder die 2-Methyl-propoxy-Gruppe bedeutet.

9. Verwendung einer Chlor-alkoxy-anilin-Verbindung von mindestens einem der Ansprüche 5 bis 8 zur Herstellung von wasserunlöslichen Azofarbstoffen, insbesondere auf der Faser, nach den Methoden der Eisfarbentechnik.

Europäisches Patentamt

# EUROPÄISCHER RECHERCHENBERICHT

Nummer der Anmeldung

EP 89 11 8141

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | FR-A- 824 338 (IG FARBENINDUSTRIE AG)<br>* Zusammenfassung; Beispiel 2 *<br>--- | 1 | C 09 B 29/00<br>D 06 P 1/12<br>D 06 P 3/68<br>C 07 C 217/84<br>C 07 C 213/00 |
| A | EP-A-0 015 557 (HOECHST AG)<br>* Ansprüche 1,2,11; Seite 8, Zeile 32 - Seite 9, Zeile 2 *<br>--- | 1,2,5,9 | |
| A | CH-A- 594 727 (ROHNER AG)<br>* Anspruch 1; Beispiel 1 *<br>--- | 1,2,4,5 | |
| A | US-A-3 617 180 (C. STRECK)<br>* Spalte 3, Zeilen 8-66; Spalte 4, Zeilen 35-54; Beispiele 27,29 *<br>----- | 1,2,4,5 | RECHERCHIERTE SACHGEBIETE (Int. Cl.5)<br>C 09 B<br>D 06 P<br>C 07 C |

Der vorliegende Recherchenbericht wurde für alle Patentansprüche erstellt

| Recherchenort | Abschlußdatum der Recherche | Prüfer |
|---|---|---|
| DEN HAAG | 08-12-1989 | GINOUX C.R.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)